# EUROPEAN PATENT SPECIFICATION

(11) **EP 1 429 716 B1**
(45) Date of publication and mention of the grant of the patent: **22.02.2006**
(21) Application number: 02747626.6
(22) Date of filing: 20.06.2002
(51) Int. Cl.: A61K 8/64, A61Q 19/08

(54) **COSMETIC PREPARATION WITH ANTI-WRINKLE ACTION**
KOSMETISCHE ZUBEREITUNG GEGEN HAUTFALTEN
PREPARATION COSMETIQUE A ACTION ANTI-RIDES

(30) Priority: 22.06.2001 CH 113601
(43) Date of publication of application: 23.06.2004
(73) Proprietor: Gecomwert Anstalt, 9490 Vaduz (LI)
(72) Inventor: GUGLIELMO, Manuela, I-30030 Vigonovo (IT); MONTANARI, Daniela, I-35100 Padova (IT)
(74) Representative: Modiano, Micaela Nadia
(86) International application number: PCT/IB2002/002392
(87) International publication number: WO 2003/000222

(56) References cited:
- PATENT ABSTRACTS OF JAPAN vol. 2000, no. 08, 6 October 2000 (2000-10-06) & JP 2000 136124 A (PIAS ARISE KK), 16 May 2000 (2000-05-16)
- PATENT ABSTRACTS OF JAPAN vol. 2000, no. 11, 3 January 2001 (2001-01-03) & JP 2000 229832 A (SHISEIDO CO), 22 August 2000 (2000-08-22)
- PATENT ABSTRACTS OF JAPAN vol. 1998, no. 01, 30 January 1998 (1998-01-30) & JP 09 241131 A (NENDO KAGAKU KENKYUSHO ET AL.), 16 September 1997 (1997-09-16)
- DATABASE WPI Week 199816 Derwent Publications Ltd., London, GB; AN 1998-174838 XP002227685 & JP 10 036283 A (ICHIMARU PHARCOS INC), 10 February 1998 (1998-02-10)
- DATABASE WPI Week 199946 Derwent Publications Ltd., London, GB; AN 1999-543933 XP002227686 & JP 11 228437 A (MICRO ALGE CORP), 24 August 1999 (1999-08-24)
- DATABASE WPI Week 197733 Derwent Publications Ltd., London, GB; AN 1977-58682y XP002227687 & JP 52 027690 B (H. OHHASHI), 21 July 1977 (1977-07-21)

## Description

The present invention relates to a cosmetic preparation in emulsion form with anti-wrinkle activity, formulated with differentiated doses of a novel active principle that is defined more clearly hereinbelow.

JP-A-2000 136 124 relates to a skin lotion capable of keeping the skin elastic, fresh and young, comprising (1) a component comprising an oligopeptide having an amino acid sequence consisting of Val-Gly- Val-Ala-Pro-Gly, a polypeptide, a protein or an extract containing the oligopeptide and/or (2) at least one extract selected from Chlorella (Chlorella), Ascophyllum (scientific name: Ascophyllum Nodosum), Silybum marianum Gaertn (scientific name: Silybum marianum (L) Gaertn) or Centella asiatica (scientific name: Centella asiatica (L) Urb).

Specifically, the present invention relates to a cosmetic composition in emulsion form, formulated as two types of emulsion, i.e. water-in-oil emulsion for a day cream, and oil-in-water emulsion for a night cream, suitable for treating skin featuring wrinkles caused by ageing. Both types of emulsion contain differentiated doses of the said active principle depending on the level of wrinkledness.

Laboratory researchers have classified wrinkles in terms of abundance and depth, creating a photographic scale of wrinkledness starting from an initial level of wrinkles, level I, and going up to a level VI with a large number of very deep wrinkles; 4 specific doses of the said active principle were thus identified for an equivalent number of levels of skin wrinkledness, from level II to V. The formulations, as day and night creams, contain doses of from 2.5 mg/g of active principle for level II wrinkles up to 5.5 mg/g of active principle for level V wrinkles.

The effects of ageing on the skin are evidenced clinically by the appearance of wrinkles, first as thin lines which then become increasingly deep, marks and the loss of tonicity and elasticity.

Histologically, the changes that occur during chronological ageing of the skin are as follows:
- thinning of the epidermis;
- reduction in the thickness of the dermis, with a decrease in the number of cells (fibroblasts) and their biosynthetic ability;
- loss of elastic tissue and decrease in collagen;
- reduction in vascularization.

Ageing is thus accompanied by a decline in cell proliferation, partly associated with a reduced response of the cells to growth factors.

This results in an overall reduction in the thickness of the skin.

The changes are not only quantitative, but also qualitative, associated with the composition and structure of the support protein fibres produced by the cells.

The preparation that is the subject matter of the invention, which is a cosmetic emulsion with anti-wrinkle action, is proposed to assist in filling in wrinkles by virtue of the presence within this preparation of an active principle that is a cellular biological reagent modified with a peptide.

The Applicant's researchers have given the novel biological reagent thus obtained the abbreviation "RB 160" and so this abbreviation will be used in the rest of the present description.

The subject of the present invention is thus a preparation with anti-wrinkle activity as described in the attached Claim 1.

The said cellular biological reagent acts where the cells are deficient, by stimulating multiplication; application of the preparation in the areas where the cells are deficient in growth factors, that is to say of low vitality in the wrinkles, allows a local increase in the amount of cells, i.e. dermal fibroblasts, to increase the thickness of the dermis.

The peptide acts on the quality of the cells by stimulating the production by the said fibroblasts of a correct extracellular matrix for the dermis, i.e. the support material in which the fibroblasts are embedded, composed of glycosaminoglycans and a three-dimensional network of collagen and elastin fibres.

The said cellular biological reagent is obtained by dissolution in water followed by sterilizing filtration of cultures of microorganisms having a very high capacity for cellular multiplication, such as: chlorella microalga, palmellococcus, chloroideum and aerosphaera.

On the basis of this discovery, the Applicant's researchers have succeeded in modifying the abovementioned biological reagent using a peptide, making it suitable for application to the skin, in the present case to a face marked with wrinkles.

The cellular biological reagent obtained as described above consists of a high content of trace elements (iron, copper, zinc and manganese) and minerals (sodium, potassium and calcium).

These trace elements and minerals are used as co-factors in various stages of metabolism: rehydration, ionic regulation, increase in all exchanges and stimulation of the natural defences.

Cellular moisturization is governed by the mechanism: absorption of sodium - release of potassium.

Sodium, potassium and chlorides represent 17% of the NMF (natural moisturization factor), essential for maintaining skin moisturization. Zinc acts as a coenzyme and takes part in enzymatic reactions for tissue regeneration.

Trace elements and minerals, acting in synergy, have an important biochemical role in regulating protein metabolism.

RB 160 is very rich in amino acids. Amino acids are primarily precursors of proteins and of essential biomolecules.

When supplied in excess relative to the requirements, they may also be used as a supply of energy to ensure the renewal of skin proteins.

60% of the amino acids contained in RB 160 are represented by essential amino acids (isoleucine, methionine, phenylalanine, threonine, tryptophan, valine, arginine and histidine).

Among these amino acids, mention should be made of the presence of a high content of arginine, a precursor of ornithine, which is in turn a precursor of spermine and spermidine, the latter being non-peptide growth factors that have the capacity of stimulating cell division and of activating protein synthesis.

Mention is also made of the high content (5% of the total) of proline, which stimulates cell turnover and regulates collagen metabolism.

Finally, the high content of water-soluble vitamins (B1, B2, B12 and PP) is involved effectively in protein synthesis. These vitamins, acting as mediators, promoting the release of energy in the form of ATP, either directly by means of the Krebs cycle (vitamin B1) or via the respiratory chain (vitamins B2 and PP). This energy is rapidly used to reintegrate protein synthesis.

Thus, vitamin B1, or thiamine, acts as a coenzyme of pyruvate dehydrogenase, which is a key enzyme in the Krebs cycle. Similarly, vitamin B12, or riboflavine, is a constituent of flavoproteins that are essential coenzymes of the respiratory chain. This is likewise the case for vitamin PP, or nicotinamide, which also acts as an energy carrier.

Vitamin B12, or cobalamine, appears to be essential for the synthesis of methionine and proteins in general. It has been demonstrated in vitro that the rate of incorporation of this amino acid in protein synthesis decreases significantly in the absence of this vitamin.

The peptide RB 160 is a lipopeptide, obtained by synthesis, having the sequence palmitoyl-Lys-Thr-Thr-Lys-Ser.

In vitro studies demonstrate that this lipopeptide induces cells to synthesize collagen and glycosaminoglycans.

The lipophilic nature of this substance increases its affinity for the skin.

RB 160 is advantageously used in the preparation that is the subject of the invention in percentages ranging from 0.25% to 4% by weight.

As anticipated, the active principle RB 160 is thus a cellular biological reagent that is capable of inducing cell multiplication, as demonstrated by in vitro tests. Using cellular cultures in sub-optimal conditions, which thus simulate a deficiency of growth factors and nutrient elements, as in the case of the cells of a wrinkled skin, to which is administered RB 160, a strong multiplication activity of the treated cells relative to the untreated cells is revealed.

The application of an emulsion containing the said cellular biological reagent RB 160 thus allows a local increase in dermal cells; by virtue of the larger number of cells present and, consequently, the larger amount of structural proteins and glycosaminoglycans produced, the said emulsion helps to make the dermis thicker and to fill in wrinkles.

The dermal activity of RB 160 is not expressed on cell cultures that are under optimum conditions, i.e. conditions that simulate those of healthy skin full of vitality.

The peptide is, specifically, a pentapeptide composed of lysine, threonine and serine, which acts specifically on dermal cells by improving their quality, that is to say their functioning, inducing them to synthesize the material necessary to support the overlying epidermis (collagen and glycosaminoglycans, components of the extracellular matrix). In particular, it induces them to produce type IV collagen, which is fundamental for the structure of the dermo-epidermal junction that holds the epidermis to the dermis.

The preparation, which may preferably be obtained in the form of an emulsion in a vehicle of gel or cream, comprises the said active principle RB 160 in a weight percentage of between 0.25% and 4%, as mentioned above.

As stated, depending on the percentage dose of the said active principle, it is possible to obtain preparations that are suitable for varying levels of wrinkledness of the skin to be treated.

## Claims

1. Cosmetic preparation with anti-wrinkle action, which may be applied externally in the form of an emulsion in a vehicle of cream or gel, comprising from 0.25% to 4% by weight of an active principle obtained from the physiological tissue of cultures of one or more microorganisms chosen from the group consisting of chlorella microalga, palmellococcus, chloroideum, aerosphaera, modified with a peptide, **characterized in that** such peptide is a pentapeptide composed of lysine, threonine and serine.

2. Preparation according to Claim 1, in which 80% by weight of the abovementioned active principle is composed of the said physiological tissue of modified chlorella microalga cultures, the remaining 20% by weight being composed of the said peptide.

3. Process for obtaining a preparation according to one of the preceding claims, in which the physiological tissue is obtained by solubilization in water followed by sterilizing filtration of chlorella microalga cultures.

## Patentansprüche

1. Kosmetische Zubereitung mit Antifaltenwirkung, die extern in Form einer Emulsion in einem Creme- oder Gelträger aufgebracht werden kann, die von 0,25 % bis 4 Gew.-% eines aktiven Prinzips umfasst, das aus dem physiologischen Gewebe von Kulturen von einem oder mehreren Mikroorganismen erhalten wurde, die aus der Gruppe ausgewählt sind, die besteht aus Chlorella-Mikroalgen, Palmellococcus, Chloroideum, Aerosphaera, das mit einem Peptid modifiziert ist, **dadurch gekennzeichnet, dass** ein solches Peptid ein Pentapeptid ist, das aus Lysin, Threonin und Serin zusammengesetzt ist.

2. Zubereitung nach Anspruch 1, bei der 80 Gew.-% des oben erwähnten aktiven Prinzips aus dem genannten physiologischen Gewebe von modifizierten Chlorella-Mikroalgen-Kulturen zusammengesetzt sind, wobei die restlichen 20 Gew.-% aus dem Peptid zusammengesetzt sind.

3. Verfahren zur Gewinnung einer Zubereitung nach irgendeinem der vorausgehenden Ansprüche, bei dem das physiologische Gewebe durch Solubilisierung in Wasser und anschließende Sterilfiltration von Chlorella-Mikroalgen-Kulturen erhalten wird.

## Revendications

1. Préparation cosmétique ayant une action antirides, qui peut être appliquée par voie externe sous forme d'une émulsion dans un véhicule de crème ou de gel, comprenant de 0,25 % à 4 % en poids d'un principe actif obtenu à partir du tissu physiologique de cultures d'un ou plusieurs micro-organismes choisis dans l'ensemble consistant en chlorella microalga, palmellococcus, chloroideum, aerosphaera, modifié par un peptide, **caractérisée en ce que** ce peptide est un pentapeptide composé de lysine, de thréonine, et de sérine.

2. Préparation selon la revendication 1, dans laquelle 80 % en poids du principe actif mentionné ci-dessus sont composés dudit tissu physiologique de cultures de chlorella microalga modifiées, les 20 % en poids restants étant composés dudit peptide.

3. Procédé pour obtenir une préparation selon l'une des revendications précédentes, dans laquelle le tissu physiologique est obtenu par solubilisation dans l'eau, suivie d'une stérilisation par filtration de cultures de chlorella microalga.
